# EUROPEAN PATENT SPECIFICATION

(11) **EP 3 517 046 B1**
(45) Date of publication and mention of the grant of the patent: **03.01.2024**
(21) Application number: 17852600.0
(22) Date of filing: 14.04.2017
(51) Int. Cl.: A61B 8/14

(54) **ULTRASONIC DIAGNOSTIC APPARATUS AND CONTROL METHOD OF ULTRASONIC DIAGNOSTIC APPARATUS**
ULTRASCHALLDIAGNOSEVORRICHTUNG UND STEUERUNGSVERFAHREN FÜR EINE ULTRASCHALLDIAGNOSEVORRICHTUNG
APPAREIL DE DIAGNOSTIC ULTRASONORE ET PROCÉDÉ DE COMMANDE D'UN APPAREIL DE DIAGNOSTIC ULTRASONORE

(30) Priority: 26.09.2016 JP 2016187535
(43) Date of publication of application: 31.07.2019
(73) Proprietor: FUJIFILM Corporation, Tokyo 106-8620 (JP)
(72) Inventor: EBATA, Tetsurou, Kanagawa 258-8538 (JP)
(74) Representative: Klunker IP Patentanwälte PartG mbB
(86) International application number: PCT/JP2017/015263
(87) International publication number: WO 2018/055819

(56) References cited:
- JP-A- H04 224 738
- JP-A- 2007 167 116
- JP-A- 2010 259 662
- JP-A- 2013 111 309
- US-A1- 2007 055 153
- US-A1- 2008 154 123
- US-A1- 2012 203 106
- US-A1- 2015 245 823
- US-A1- 2015 327 841

## Description

### BACKGROUND OF THE INVENTION

### 1. Field of the Invention

The present invention relates to an ultrasound diagnostic apparatus and a control method of the ultrasound diagnostic apparatus, and more particularly, to an ultrasound diagnostic apparatus that discriminates an inspection portion that is currently being imaged.

### 2. Description of the Related Art

In the related art, an ultrasound diagnostic apparatus that uses an ultrasound image has been put to practical use in a medical field. Generally, such an ultrasound diagnostic apparatus operates an ultrasound beam into a subject from an ultrasound probe in which an array transducer is provided, receives an ultrasound echo from the subject using the ultrasound probe to output a reception signal, and electrically processes the reception signal to generate an ultrasound image.

In a case where a plurality of inspection portions of the subject are diagnosed using such an ultrasound image, in order to obtain ultrasound images suitable for diagnosis with respect to the respective inspection portions, it is necessary to set different appropriate imaging conditions in accordance with the inspection portions. In this regard, for example, JP-H4-224738A (JP1992-224738A) discloses an ultrasound diagnostic apparatus that automatically discriminates an inspection portion from a generated ultrasound image through a pattern matching process and sets imaging conditions suitable for the inspection portion on the basis of the discrimination result.

US2008/154123A1 discloses techniques for automated image interpretation with transducer position or orientation sensing for medical ultrasound. Automated image interpretation is provided with transducer position and/or orientation sensing. The current position of a transducer is used to determine the anatomy being imaged. Other reference information, such as (1) the previous position of the transducer and the known anatomy being imaged at that time or (2) a reference device at a known location, is used in the determination. The anatomy is determined based on position sensing or determined based on position sensing and other anatomy features, such as image analysis.

US2015/245823A1 discloses a wireless probe and method for power controlling of wireless probe. A wireless probe includes: an ultrasound reception/transmission module that receives a first power and scans a target object by transmitting ultrasound signals to the target object and receiving ultrasound echo signals reflected from the target object; a signal processing module that receives a second power, generates pulses for generating the ultrasound signals and generates ultrasound data by using the ultrasound echo signals; a wireless communication module that receives a third power and receives/transmits predetermined data from/to a medical device; a controller that controls supply of at least one of the first power, the second power, and the third power based on an operation state of the wireless probe; and a power unit that supplies or blocks the at least one of the first power, the second power, and the third power according to control of the controller.

### SUMMARY OF THE INVENTION

However, since an ultrasound image is changed due to various causes such as a difference between shapes of inspection portions and a difference between dynamic ranges or brightnesses due to a difference between passage easinesses of ultrasound for the inspection portions, there is a concern that the inspection portions may be mistakenly discriminated only using the discrimination of the inspection portions based on the ultrasound image. In this case, there is a concern that inappropriate imaging conditions may be set on the basis of the mistaken discrimination result and an ultrasound image with a low image quality may be generated to cause an error in diagnosis.

The invention has been made in consideration of the problems in the related art, and an object of the invention is to provide an ultrasound diagnostic apparatus and a control method of the ultrasound diagnostic apparatus capable of accurately discriminating an inspection portion.

According to an aspect of the invention, there is provided an ultrasound diagnostic apparatus according to claim 1 of the appended claims.

The portion discrimination section may integrate the image analysis result in the image analysis section and the movement direction and the movement distance calculated by the movement amount calculation section to discriminate the second inspection portion.

Further, the portion discrimination section may narrow down the plurality of inspection portions that are targets of the image analysis, on the basis of the movement direction and the movement distance calculated by the movement amount calculation section, and the image analysis section may perform the image analysis with respect to the inspection portions narrowed down by the portion discrimination section, and the portion discrimination section may discriminate the second inspection portion using the image analysis result in the image analysis section.

Further, the portion discrimination section may determine an analysis order for performing the image analysis with respect to the plurality of inspection portions, on the basis of the movement direction and the movement distance calculated by the movement amount calculation section, the image analysis section may sequentially perform the image analysis with respect to the plurality of inspection portions in accordance with the analysis order determined by the portion discrimination section, and the portion discrimination section may discriminate the second inspection portion using the image analysis result in the image analysis section.

The ultrasound diagnostic apparatus further comprises: a movement amount reference value memory in which a plurality of movement amount reference values relating to the movement direction and the movement distance of the ultrasound probe in a case where the ultrasound probe is moved between the plurality of inspection portions are stored in advance. The portion discrimination section may read out the plurality of movement amount reference values from the movement amount reference value memory, compares each of the plurality of read-out movement amount reference values with the movement direction and the movement distance calculated by the movement amount calculation section, and may discriminate the second inspection portion on the basis of the comparison result and the image analysis result in the image analysis section.

Further, it is preferable that the portion discrimination section corrects the plurality of movement amount reference values in accordance with differences between the movement direction and the movement distance calculated by the movement amount calculation section and used when the second inspection portion is discriminated and the movement amount reference values used when the second inspection portion is discriminated, and uses the plurality of corrected movement amount reference values in discriminating an inspection portion that becomes a next inspection target subsequent to the second inspection portion.

The ultrasound diagnostic apparatus may further comprise: a subject reference value memory in which a plurality of subject reference values relating to the movement direction and the movement distance in a case where the ultrasound probe is moved between the plurality of inspection portions for each subject are stored in advance. The portion discrimination section may read out the subject reference values corresponding the subject from the subject reference value memory, may compare the read-out subject reference values with the movement direction and the movement distance calculated by the movement amount calculation section, and may discriminate the second inspection portion on the basis of the comparison result and the image analysis result in the image analysis section.

The movement amount calculation section detects an acceleration of the ultrasound probe using the detection signal output from the movement detection sensor, and may calculate the movement direction and the movement distance of the ultrasound probe from a time when the detected acceleration becomes equal to or larger than a predetermined threshold value to a time when the detected acceleration becomes smaller than the predetermined threshold value.

Further, the ultrasound diagnostic apparatus may further comprise: a probe state determination section that determines whether the ultrasound probe is in an air radiation state or in a contact state with respect to the subject. The movement amount calculation section may calculate the movement direction and the movement distance of the ultrasound probe from a time when it is determined by the probe state determination section that the ultrasound probe transitions from the contact state with respect to the subject to the air radiation state to a time when it is determined by the probe state determination section that the ultrasound probe transitions from the air radiation state to the contact state with respect to the subject.

The movement detection sensor is formed by an acceleration sensor.

According to an aspect of the invention, there is provided a control method according to claim 9 of the appended claims.

According to the invention, since the ultrasound diagnostic apparatus that sequentially inspects a plurality of inspection portions of a subject comprises an ultrasound probe; an imaging section that performs transmission and reception of an ultrasound beam between a subject and the ultrasound probe and generates an ultrasound image on the basis of a reception signal output from the ultrasound probe; an image analysis section that performs image analysis using the ultrasound image generated by the imaging section; a movement detection sensor that is attached to the ultrasound probe and detects a movement of the ultrasound probe to output the movement as a detection signal; a movement amount calculation section that calculates a movement direction and a movement distance of the ultrasound probe in a case where the ultrasound probe is moved from a first inspection portion where inspection is terminated among the plurality of inspection portions to a second inspection portion that is the next inspection target, using the detection signal output from the movement detection sensor; and a portion discrimination section that discriminates the second inspection portion on the basis of an image analysis result in the image analysis section and the movement direction and the movement distance calculated by the movement amount calculation section, it is possible to accurately discriminate an inspection portion.

### BRIEF DESCRIPTION OF THE DRAWINGS

Fig. 1 is a diagram showing a configuration of ultrasound diagnostic apparatus according to Embodiment 1 of the invention.
Fig. 2 is a diagram showing a configuration of a reception section.
Fig. 3 is a diagram showing a configuration of an image processing section.
Fig. 4 is a diagram showing an example of a movement direction and a movement distance of an ultrasound probe.
Fig. 5 is a flowchart showing an operation of Embodiment 1.
Fig. 6 is a flowchart showing operations of an image generation process and a movement detection process.
Fig. 7 is a diagram showing an example of an ultrasound image of a left lung.
Fig. 8 is a diagram showing an example of an ultrasound image of a left abdomen.
Fig. 9 is a diagram showing a configuration of ultrasound diagnosis apparatus according to Embodiment 5.
Fig. 10 is a diagram showing a configuration of ultrasound diagnosis apparatus according to Embodiment 6.

### DESCRIPTION OF THE PREFERRED EMBODIMENTS

Hereinafter, embodiments of the invention will be described on the basis of the accompanying drawings.

### Embodiment 1

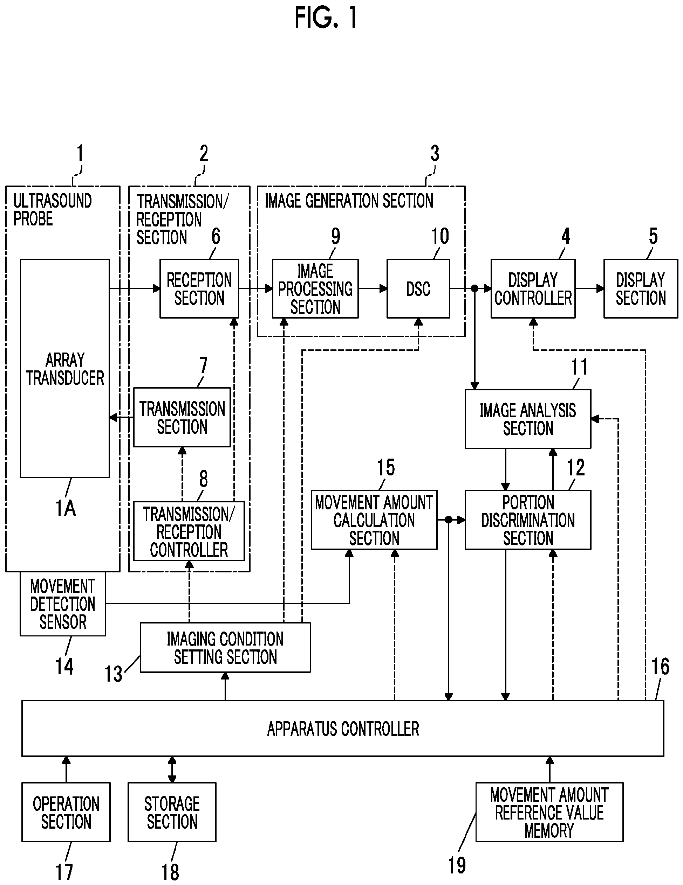

Fig. 1 shows a configuration of an ultrasound diagnostic apparatus according to Embodiment 1. The ultrasound diagnostic apparatus comprises an ultrasound probe 1 in which an array transducer 1A is provided, an image generation section 3 that is connected to the ultrasound probe 1 through a transmission/reception section 2, and a display section 5 that is connected to the image generation section 3 through a display controller 4.

The transmission/reception section 2 includes a reception section 6 and a transmission section 7 that are connected to the array transducer 1A, and a transmission/reception controller 8 that is connected to the reception section 6 and the transmission section 7. The image generation section 3 includes an image processing section 9 and a digital scan converter (DSC) 10 that is connected to the image processing section 9. The display controller 4 is connected to the DSC 10. Further, an image analysis section 11 is connected to the DSC 10, and a portion discrimination section 12 is connected to the image analysis section 11.

An imaging condition setting section 13 is connected to the transmission/reception controller 8 of the transmission/reception section 2, and the image processing section 9 and the DSC 10 of the image generation section 3.

A movement detection sensor 14 is attached to the ultrasound probe 1, and a movement amount calculation section 15 is connected to the movement detection sensor 14. Further, the portion discrimination section 12 is also connected to the movement amount calculation section 15.

An apparatus controller 16 is connected to the display controller 4, the image analysis section 11, the portion discrimination section 12, the imaging condition setting section 13, and the movement amount calculation section 15. Further, an operation section 17, a storage section 18, and a movement amount reference value memory 19 are respectively connected to the apparatus controller 16.

The array transducer 1A of the ultrasound probe 1 includes a plurality of ultrasound transducers that are arranged in one dimension or two dimensions. Each of the ultrasound transducers transmits ultrasound in accordance with a drive signal supplied from the transmission section 7, and receives an ultrasound echo from a subject to output a reception signal. Each ultrasound transducer is formed using a vibrator in which electrodes are formed on opposite ends of a piezoelectric body formed of piezoelectric ceramics represented as lead zirconate titanate (PZT), a high polymer piezoelectric element represented as polyvinylidene fluoride (PVDF), piezoelectric crystals represented as magnesium niobate-lead titanate solute (PMN-PT), or the like.

In a case where a pulse-shaped voltage or a continuous wave voltage is applied to the electrodes of the vibrator, the piezoelectric body expands and contracts, a pulse-shaped ultrasound or a continuous wave ultrasound is generated from each vibrator, and an ultrasound beam is formed by synthesis of the ultrasounds. Further, each vibrator receives a propagating ultrasound to stretch and compresses to generate an electric signal, and the electric signal is output as an ultrasound reception signal.

The transmission/reception section 2 performs transmission and reception of an ultrasound beam in accordance with a set ultrasound beam scanning condition, and the image generation section 3 generates an ultrasound image signal in accordance with the set ultrasound image generation condition. The transmission/reception section 2 and the image generation section 3 form an imaging section.

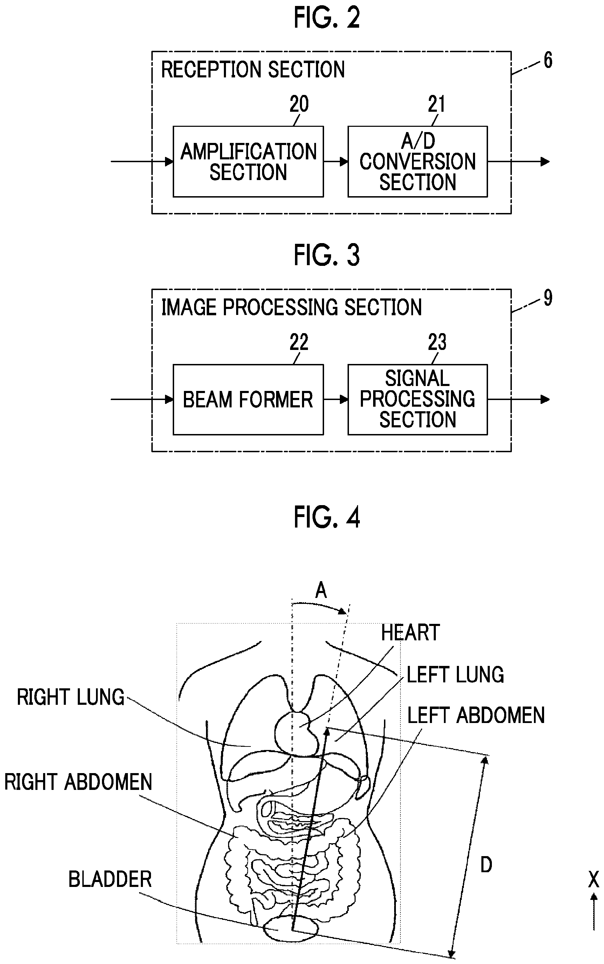

The reception section 6 of the transmission/reception section 2 has a configuration in which an amplification section 20 and an analogue/digital (A/D) conversion section 21 are sequentially connected in series, as shown in Fig. 2. The reception section 6 amplifies a reception signal transmitted from each ultrasound transducer of the array transducer 1A using the amplification section 20, and performs A/D conversion with respect to the amplified signal using the A/D conversion section 21 to generate digital reception data.

The transmission/reception controller 8 controls the reception section 6 and the transmission section 7 so that transmission of ultrasound pulses to a subject and reception of ultrasound echoes from the subject are repeated at a pulse repetition frequency (PRF) interval, on the basis of various control signals transmitted from the apparatus controller 16.

The image processing section 9 of the image generation section 3 has a configuration in which a beam former 22 and a signal processing section 23 are sequentially connected in series, as shown in Fig. 3. The beam former 22 assigns a delay to each piece of reception data output from the reception section 6 of the transmission/reception section 2 in accordance with sound velocities set on the basis of a reception delay pattern selected in accordance with control signals from the imaging condition setting section 13 or a distribution of the sound velocities and adds up the results to perform a reception focus process. Through the reception focus process, a sound ray signal in which focuses of ultrasound echoes after phasing addition are narrowed down is generated.

The signal processing section 23 corrects attenuation due to a distance in accordance with a depth of a reflecting position of ultrasound with respect to a sound ray signal generated by the beam former 22, and then, performs an envelope detection process and performs a variety of necessary image processing such as a gradation process, to thereby generate an ultrasound image signal that is tomographic image information of a tissue in a subject.

As the ultrasound image, for example, a brightness mode (B mode) image, a motion mode (M mode) image, a color Doppler imaging, or the like may be used Further, a sound velocity map indicating a distribution of sound velocities, or an elasticity map indicating a distribution of elasticities indicating smoothness or the like of a tissue in a subject may be used as the ultrasound image.

The DSC 10 of the image generation section 3 converts an ultrasound image signal generated by the signal processing section 23 of the image processing section 9 into an image signal based on a scanning method of a general television signal (raster conversion).

The display section 5 includes a display device such as a liquid crystal display (LCD), for example, and displays an ultrasound image under the control of the display controller 4.

The image analysis section 11 performs image analysis using an ultrasound image from the DSC 10, and outputs the image analysis result to the portion discrimination section 12. For example, a feature of the ultrasound image such as a brightness or an edge of the ultrasound image is detected. Further, in a case where a B mode image signal or an M mode image signal is used, the image analysis may be performed on the basis of a known pattern recognition method such as machine learning, template matching, or texture analysis. In addition, in a case where a color Doppler image signal, a sound velocity map or an elasticity map is used, the image analysis may be performed on the basis of a known method such as color information analysis.

The movement detection sensor 14 is attached to the ultrasound probe 1, and detects a movement of the ultrasound probe 1 in a case where the ultrasound probe 1 is operated by an operator and outputs the movement of the ultrasound probe 1 to the movement amount calculation section 15 as a detection signal. The movement detection sensor 14 is an an acceleration sensor. Further, in order to more accurately detect the movement of the ultrasound probe 1, plural sensors among a gyro sensor, a magnetic sensor a GPS sensor, or other sensors capable of detecting a movement may be used in combination.

As inspection portions of the subject, for example, in a case where an extended focused assessment with sonography for trauma (eFAST) inspection for sequentially inspecting a plurality of inspection portions is considered, the left lung, the right lung, the heart, the left abdomen, the right abdomen, and the bladder, and the like may be used. Portions other than the plurality of inspection portions may be added. Here, among the plurality of inspection portions of the subject, an inspection portion for which inspection is terminated is defined as a first inspection portion. Further, an inspection portion that becomes a next inspection target subsequent to the first inspection portion is defined as a second inspection portion. The ultrasound probe 1 is moved from the first inspection portion for which the inspection is terminated to the second inspection portion that becomes the next inspection target, through an operation of an operator. Further, the second inspection portion is imaged, and an ultrasound image is generated.

The movement amount calculation section 15 calculates a movement direction and a movement distance of the ultrasound probe 1 in a case where the ultrasound probe 1 is moved from the first inspection portion to the second inspection portion using a detection signal from the movement detection sensor 14, and outputs the result to the portion discrimination section 12. For example, as shown in Fig. 4, it is assumed that the first inspection portion of the subject is the bladder and the second inspection portion that is the next inspection target is the left lung. Here, for ease of description, a direction that is directed from the bladder to the head is defined as an X direction. The movement amount calculation section 15 calculates a movement distance D in a case where the ultrasound probe 1 is moved from the bladder to the left lung, and calculates an angle A in a clockwise direction with reference to the X direction. In this way, the movement direction and the movement distance in a case where the ultrasound probe 1 is moved from the bladder to the left lung are calculated.

Since it is considered that between inspection of the first inspection portion and inspection of the second inspection portion, a gel for filling a gap between the subject and the ultrasound probe 1 is assigned, or the ultrasound probe 1 is once placed, it is preferable that the movement distance D is calculated as a linear distance.

The movement amount reference value memory 19 stores in advance a plurality of movement amount reference values relating to a movement direction and a movement distance of the ultrasound probe 1 in a case where the ultrasound probe 1 is moved between a plurality of inspection portions. For example, as shown in Table 1, with respect to a subject who has an average body type, a plurality of movement amount reference values relating to a movement direction and a movement distance of the ultrasound probe 1 in a case where the ultrasound probe 1 is moved between a plurality of inspection portions such as the left lung, the right lung, the heart, the left abdomen, the right abdomen, and the bladder are stored.

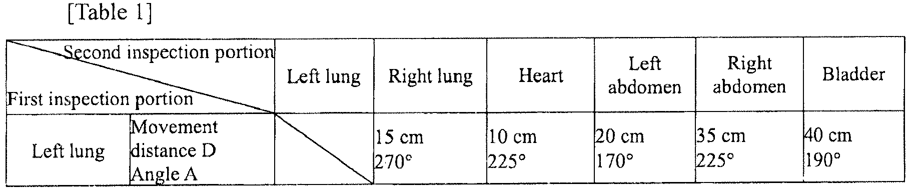

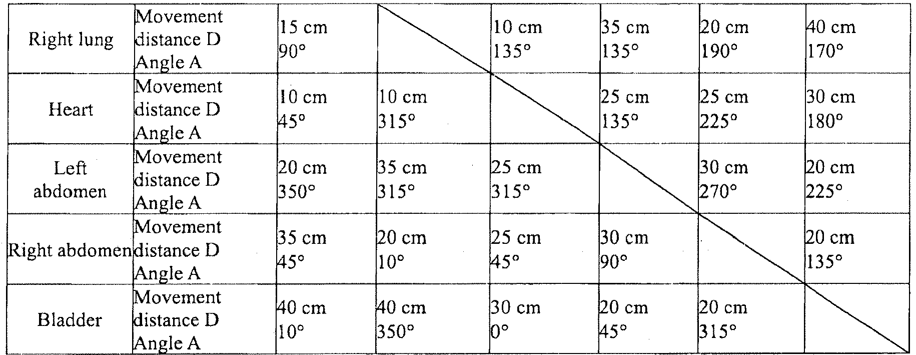

The portion discrimination section 12 discriminates the second inspection portion that is currently being imaged, on the basis of the image analysis result in the image analysis section 11 and the movement direction and the movement distance of the ultrasound probe 1 calculated by the movement amount calculation section 15, and outputs the portion discrimination results to the apparatus controller 16.

Specifically, the portion discrimination section 12 reads out a plurality of movement amount reference values from the movement amount reference value memory 19, and compares the plurality of read-out movement amount reference values with the movement direction and the movement distance of the ultrasound probe 1 calculated by the movement amount calculation section 15, respectively. Further, the portion discrimination section 12 combines the comparison result and the image analysis result in the image analysis section 11 to discriminate the second inspection portion. In order to perform the portion discrimination, for example, a support vector machine (SVM) algorithm, a decision tree algorithm, or other known discrimination algorithms may be used.

In this way, the portion discrimination section 12 may integrate the image analysis result and the movement direction and the movement distance of the ultrasound probe 1 to perform the portion discrimination.

The apparatus controller 16 outputs the portion discrimination result output from the portion discrimination section 12 to the imaging condition setting section 13.

Further, the apparatus controller 16 controls the display controller 4, the image analysis section 11, the portion discrimination section 12, the imaging condition setting section 13, and the movement amount calculation section 15 on the basis of commands input through the operation section 17 from the operator.

The imaging condition setting section 13 sets imaging conditions suitable for the discriminated second inspection portion with respect to the imaging section formed by the transmission/reception section 2 and the image generation section 3, on the basis of the portion discrimination result input from the apparatus controller 16. The imaging conditions include an ultrasound beam scanning condition for the transmission/reception section 2 and an ultrasound image generation condition for the image generation section 3.

Among the imaging conditions, as the ultrasound beam scanning condition for the transmission/reception section 2, a transmission frequency of an ultrasound beam, a focal position, a display depth, or the like may be used, and as the ultrasound image generation condition for the image generation section 3, a sound velocity, a wave detection condition, a gain, a dynamic range, a gradation curve, a speckle suppression strength, an edge emphasis degree, or the like may be used.

The operation section 17 is a unit through which an operator performs an input operation, and may be formed by a keyboard, a mouse, a trackball, a touch panel, or the like.

The storage section 18 stores an operation program or the like, and may be configured using a recording medium such as a hard disk, a flexible disc, a magneto-optical disc (MO), a magnetic tape (MT), a random access memory (RAM), a compact disc read only memory (CD-ROM), a digital versatile disc read only memory (DVD-ROM), a secure digital card (SD card), a compact flash card (CF card), a universal serial bus memory (USB memory), or a server.

The transmission/reception controller 8 of the transmission/reception section 2, the image generation section 3, the display controller 4, the image analysis section 11, the portion discrimination section 12, the imaging condition setting section 13, the movement amount calculation section 15, and the apparatus controller 16 are configured by a central processing unit (CPU), and an operation program for causing the CPU to execute various processes, and may be configured by a digital circuit. Further, a configuration in which the transmission/reception controller 8 of the transmission/reception section 2, the image generation section 3, the display controller 4, the image analysis section 11, the portion discrimination section 12, the imaging condition setting section 13, the movement amount calculation section 15, and the apparatus controller 16 are partially or generally integrated into one CPU may be employed.

Here, a method for discriminating the first inspection portion will be described.

The first inspection portion is discriminated by the portion discrimination section 12 using an image analysis result. That is, the image analysis section 11 performs image analysis with respect to an ultrasound image signal from the DSC 10, and outputs the image analysis result to the portion discrimination section 12. Further, the portion discrimination section 12 discriminates the first inspection portion using a feature of an ultrasound image such as a brightness or an edge detected by the image analysis.

Alternatively, the first inspection portion may be discriminated by inputting information indicating which inspection portion the first inspection portion is through the operation section 17 from an operator.

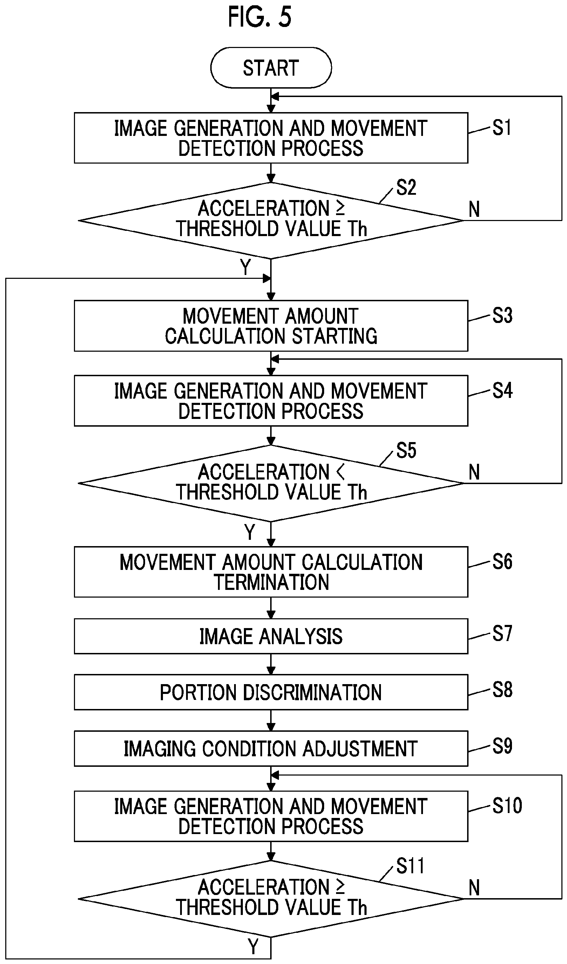

Next, an operation of Embodiment 1 will be described with reference to a flowchart of Fig. 5.

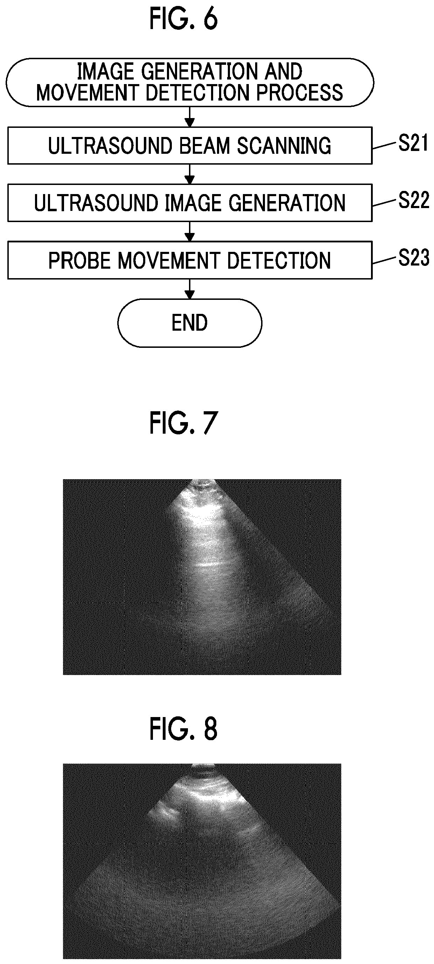

First, in step S1, an image generation process and a movement detection process are performed. Specifically, in accordance with a flowchart shown in Fig. 6, an ultrasound image is generated, and a movement of the ultrasound probe 1 is detected. In step S21, transmission and reception and scanning of an ultrasound beam using the plurality of ultrasound transducers of the array transducer 1A of the ultrasound probe 1 are performed by the transmission/reception section 2, a reception signal is output to the reception section 6 from each ultrasound transducer that receives an ultrasound echo from a subject, and is amplified and A/D converted in the reception section 6 to generate reception data.

Then, in step S22, the reception data is input to the image generation section 3, and is subjected to a reception focus process in the image processing section 9. Then, the data is subjected to image conversion in the DSC 10 to generate an ultrasound image signal. The ultrasound image signal is output to the display controller 4 from the image generation section 3, so that an ultrasound image is displayed on the display section 5. Here, for example, in a case where the first inspection portion is being inspected, the ultrasound image of the first inspection portion is displayed on the display section 5. Further, the ultrasound image signal is also output to the image analysis section 11.

Further, in step S23, a movement of the ultrasound probe 1 in a case where the ultrasound probe 1 is operated by the operator is detected by the movement detection sensor 14 attached to the ultrasound probe 1, and is output to the movement amount calculation section 15 as a detection signal. According to the invention, an acceleration sensor is attached as the movement detection sensor 14 and an acceleration is output to the movement amount calculation section 15 as a detection signal.

Further, in step S2 in the flowchart shown in Fig. 5, it is determined by the movement amount calculation section 15 whether the detected acceleration is equal to or greater than a predetermined threshold value Th, and the determination result is output to the apparatus controller 16. In a case where it is determined that the detected acceleration is smaller than the threshold value Th, it is considered that the inspection of the first inspection portion is not terminated, step S1 and step S2 are repeated so that the inspection of the first inspection portion is continued.

In step S2, in a case where it is determined that the detected acceleration is equal to or greater than the threshold value Th, it is considered that the inspection of the first inspection portion is terminated so that the ultrasound probe 1 begins moving from the first inspection portion to the second inspection portion that is the next inspection target, and then, the procedure proceeds to step S3.

In the subsequent step S3, calculation of a movement amount of the ultrasound probe 1 is started using the movement amount calculation section 15. Further, in step S4, an ultrasound image is generated in accordance with the flowchart shown in Fig. 6. Then, a movement of the ultrasound probe 1, that is, an acceleration is detected, and the acceleration is used for calculation of a movement direction and a movement distance of the ultrasound probe 1.

In step S5 of the flowchart shown in Fig. 5, it is determined by the movement amount calculation section 15 whether the detected acceleration is smaller than the threshold value Th used in step S2, and the determination result is output to the apparatus controller 16. In a case where it is determined that the detected acceleration is equal to or greater than the threshold value Th, it is considered that the ultrasound probe 1 is being moved, so that step S4 and step S5 are repeated until the ultrasound probe 1 is in contact with a body surface S, and the calculation of the movement direction and the movement distance of the ultrasound probe 1 is continued.

In step S5, in a case where it is determined that the detected acceleration is smaller than the threshold value Th, it is considered that the movement of the ultrasound probe 1 is completed and the ultrasound probe 1 is in contact with the body surface S so that the second inspection portion is imaged. Then, the procedure proceeds to step S6.

In step S6, the calculation of the movement amount of the ultrasound probe 1 is terminated by the movement amount calculation section 15, and calculation results of the movement direction and the movement distance of the ultrasound probe 1 are output to the portion discrimination section 12.

In the subsequent step S7, image analysis is performed by the image analysis section 11 using the ultrasound image. The image analysis section 11 detects a feature of the ultrasound image such as a brightness or an edge of the ultrasound image output from the image generation section 3, and outputs the image analysis result to the portion discrimination section 12.

In step S8, the second inspection portion that is currently being imaged is discriminated by the portion discrimination section 12. The portion discrimination section 12 reads out a plurality of movement amount reference values from the movement amount reference value memory 19, and compares the read-out movement amount reference values with the calculation results calculated by the movement amount calculation section 15, respectively. Further, the portion discrimination section 12 integrates the comparison result and the image analysis result output from the image analysis section 11, discriminates the second inspection portion from the plurality of inspection portions, and outputs the portion discrimination result to the apparatus controller 16.

Further, in step S9, the portion discrimination result in the portion discrimination section 12 is output to the imaging condition setting section 13 through the apparatus controller 16. Further, the imaging condition setting section 13 sets imaging conditions based on the portion discrimination result, and controls the transmission/reception section 2 and the image generation section 3 on the imaging conditions.

In the next step S10, in accordance with the flowchart shown in Fig. 6, an ultrasound image is generated on the basis of the imaging conditions set by the imaging condition setting section 13, and the movement of the ultrasound probe 1, that is, the acceleration is detected.

In step S11, it is determined by the movement amount calculation section 15 whether the detected acceleration is equal to or greater than the threshold value Th used in step S2. In a case where it is determined that the detected acceleration is smaller than the threshold value Th, since it is considered that the second inspection portion is being diagnosed, the procedure returns to step S10. Then step S10 and step S11 are repeated, so that the diagnosis is continued.

On the other hand, in step S11, in a case where it is determined that the detected acceleration is equal to or greater than the threshold value Th, since it is considered that inspection of the left lung that is the second inspection portion is terminated so that the ultrasound probe 1 is moved to an inspection portion that is a next inspection target subsequent to the second inspection portion, the procedure returns to step S3. Then, through steps S3 to S9, the inspection portion that is the next inspection target subsequent to the second inspection portion is discriminated, imaging conditions based on the discriminated inspection portion are set, and step S10 and step S11 are repeated, so that the diagnosis may be continued.

Next, an example of portion discrimination using the portion discrimination section 12 in a case where it is discriminated that the left lung, the right lung, the heart, the left abdomen, the right abdomen, and the bladder are inspection portions and the first inspection portion is the bladder will be described.

It is assumed that calculation results of a movement direction and a movement distance of the ultrasound probe 1, of a movement distance D of 35 cm and an angle A of 15° are obtained by the movement amount calculation section 15. When comparing the calculation results of the movement direction and the movement distance of the ultrasound probe 1 with the movement amount reference values written in Table 1, respectively, the calculation results show values close to movement amount reference values between the bladder and the left lung and between the bladder and the heart. On the other hand, the calculation results show values that are quite different from movement amount reference values between the bladder and the right lung, between the bladder and the left abdomen, and between the bladder and the right abdomen. Thus, it is determined that any one of the left lung and the heart is the second inspection portion. However, it is not possible to determine which one of the left lung and the heart is the second inspection portion only using the comparison result.

On the other hand, since similar structures with respect to the left lung and the left abdomen are shown in a feature of the ultrasound image such as a brightness or an edge obtained by the image analysis section 11, any one of the left lung and the left abdomen is determined as the second inspection portion. For example, an example of an ultrasound image of the left lung shown in Fig. 7 is similar to an example of an ultrasound image of the left abdomen shown in Fig. 8. However, it is not possible to determine which one of the left lung and the left abdomen is the second inspection portion only using the image analysis result.

Accordingly, by integrating the calculation results of the movement direction and the movement distance of the ultrasound probe 1 and the image analysis result, it is possible to discriminate that the second inspection portion is the left lung.

In this way, even in a case where it is difficult to perform portion discrimination only using an image analysis result or calculation results of a movement direction and a movement distance of the ultrasound probe 1, it is possible to integrate the calculation results of the movement direction and the movement distance of the ultrasound probe 1 and the image analysis result to thereby accurately discriminate the second inspection portion.

### Embodiment 2

In Embodiment 1, the image analysis result and the movement direction and the movement distance of the ultrasound probe 1 are integrated to discriminate the second inspection portion, but in Embodiment 2, a plurality of inspection portions that are image analysis targets are narrowed down on the basis of the movement direction and the movement distance of the ultrasound probe 1.

Through steps S1 to S6 in the flowchart shown in Fig. 5, an ultrasound image is generated, and a movement direction and a movement distance of the ultrasound probe 1 in a case where the ultrasound probe 1 is moved from a first inspection portion to a second inspection portion that is the next inspection target are calculated by the movement amount calculation section 15.

Further, in step S7, in a case where image analysis is performed by the image analysis section 11 using the ultrasound image, a plurality of inspection portions that are targets of the image analysis are narrowed down by the portion discrimination section 12. The portion discrimination section 12 reads out a plurality of movement amount reference values shown in Table 1 from the movement amount reference value memory 19, and sets separation allowances with respect to the plurality of movement amount reference values. Further, in a case where calculation results of the movement direction and the movement distance of the ultrasound probe 1 are within ranges of the separation allowances, a corresponding inspection portion is set as an image analysis target, and in a case where the calculation results are out of the ranges of the separation allowances, the corresponding inspection portion is excluded from the image analysis target. In this way, the plurality of inspection portions that are the image analysis targets are narrowed down, and the narrowing down result is output to the image analysis section 11.

Further, the image analysis section 11 performs image analysis with respect to the plurality of inspection portions that are narrowed down by the portion discrimination section 12, and outputs the image analysis result to the portion discrimination section 12. Further, in step S8, the portion discrimination section 12 discriminates the second inspection portion using the image analysis result.

In this way, by narrowing down a plurality of inspection portions that are targets of image analysis on the basis of a movement direction and a movement distance of the ultrasound probe 1, it is possible to reduce the number of image analysis processes for which it is generally considered that a processing load is high, and to effectively reduce the processing load due to the image analysis processes.

Next, an example of narrowing down of inspection portions in the portion discrimination section 12 in a case where the left lung, the right lung, the heart, the left abdomen, the right abdomen, and the bladder are inspection portions and it is discriminated that the first inspection portion is the bladder will be described.

It is assumed that calculation results of a movement direction and a movement distance of the ultrasound probe 1, of a movement distance D of 35 cm and an angle A of 15° are obtained by the movement amount calculation section 15. With respect to the plurality of movement amount reference values shown in Table 1, a separation allowance of the movement distance D is set to ±10 cm, and a separation allowance of the angle A is set to ±20°. In a case where the separation allowances are set, different values may be set for each inspection portion.

For example, in a case where the separation allowance of the movement distance D between the bladder and the left lung is 30 cm to 50 cm and the separation allowance of the angle A is -10° to 30°, the calculation results of the movement direction and the movement distance of the ultrasound probe 1 are within ranges of the separation allowances. Similarly, the calculation results are within ranges of the separation allowances between the bladder and the heart. On the other hand, the calculation results are out of the ranges of the separation allowances between the bladder and the right lung, between the bladder and the left abdomen, and between the bladder and the right abdomen. The image analysis targets are narrowed down to the left lung and the heart on the basis of the comparison results.

It may be considered that, for each body type of a subject, for example, according to whether the subject is an adult or a child, a direction variation between inspection portions is small and a distance variation between the inspection portions is large. Thus, it may be considered that the variation of the ultrasound probe 1 in the movement direction becomes small and the variation of the ultrasound probe 1 in the movement distance becomes large. Accordingly, it is possible to narrow down the plurality of inspection portions that are targets of image analysis on the basis of only the movement direction of the ultrasound probe 1.

Further, the portion discrimination section 12 may integrate the image analysis results for the plurality of inspection portions that are narrowed down and the movement direction and the movement distance of the ultrasound probe 1 calculated by the movement amount calculation section 15 to discriminate the second inspection portion. By narrowing down the inspection portions, it is possible to rapidly and accurately discriminate the second inspection portion while reducing a processing load due to the image analysis process.

Further, in narrowing down the inspection portions, in order to prevent inspection portions from being overlooked, there is a case where the separation allowances are set to large values to some extent, and thus, the inspection portions may not be sufficiently narrowed down. However, even in a case where the inspection portions are not sufficiently narrowed down, it is possible to accurately discriminate the second inspection portion by integrating the image analysis results with the movement direction and the movement distance of the ultrasound probe 1.

### Embodiment 3

In Embodiment 1 and Embodiment 2, an analysis order for performing image analysis with respect to a plurality of inspection portions is not fixed, but in Embodiment 3, the analysis order is determined on the basis of a movement direction and a movement distance of the ultrasound probe 1.

Through steps S1 to S6 in the flowchart of Fig. 5, an ultrasound image is generated, and a movement direction and a movement distance of the ultrasound probe 1 are calculated in a case where the ultrasound probe 1 is moved from a first inspection portion to a second inspection portion that is the next inspection target by the movement amount calculation section 15.

In the next step S7, in a case where image analysis is performed by the image analysis section 11 using the ultrasound image, an analysis order for performing the image analysis with respect to a plurality of inspection portions is determined by the portion discrimination section 12. The portion discrimination section 12 reads out a plurality of movement amount reference values shown in Table 1 from the movement amount reference value memory 19, and compares the read-out movement amount reference values with the movement direction and the movement distance of the ultrasound probe 1 calculated by the movement amount calculation section 15, respectively. As the calculation results of the movement direction and the movement distance of the ultrasound probe 1 are respectively closer to the movement amount reference values, the portion discrimination section 12 increases a priority of image analysis with respect to a corresponding inspection portion, and as the calculation results are respectively more distant from the movement amount reference values, the portion discrimination section 12 decreases a priority of image analysis for a corresponding inspection portion. In this way, the analysis order is determined with respect to the plurality of inspection portions.

Further, the image analysis section 11 sequentially performs image analysis with respect to the plurality of inspection portions in accordance with the analysis order determined by the portion discrimination section 12, and outputs the image analysis result to the portion discrimination section 12. Further, in step S8, the portion discrimination section 12 discriminates the second inspection portion using the image analysis result. Here, the image analysis is preferentially performed with respect to an inspection portion for which it is determined in advance that there is a high possibility that the inspection portion corresponds to the second inspection portion on the basis of the movement direction and the movement distance of the ultrasound probe 1. Thus, a possibility that it is discriminated that the second inspection portion corresponds to an inspection portion that is a target of preferential image analysis becomes high.

Next, an example of determination of an analysis order using the portion discrimination section 12 in a case where the left lung, the right lung, the heart, the left abdomen, the right abdomen, and the bladder are inspection portions and it is discriminated that the first inspection portion is the bladder will be described.

It is assumed that calculation results of a movement direction and a movement distance of the ultrasound probe 1, of a movement distance D of 35 cm and an angle A of 15° are obtained by the movement amount calculation section 15. When comparing the calculation results of the movement direction and the movement distance of the ultrasound probe 1 with the plurality of movement amount reference values written in Table 1, respectively, the calculation results show close values in the order of movement amount reference values between the bladder and the left lung, between the bladder and the heart, between the bladder and the right lung, between the bladder and the left abdomen, and the bladder and the right abdomen,. Through the comparison results, the analysis order is determined so that targets of image analysis are set in the order of the left lung, the heart, the right lung, the left abdomen, and the right abdomen.

In this way, by determining an analysis order for performing image analysis with respect to a plurality of inspection portions, it is possible to discriminate the second inspection portion in a short time, and thus, it is possible to enhance response performance of the ultrasound diagnostic apparatus according to Embodiment 3.

### Embodiment 4

In Embodiments 1 to 3, movement amount reference values relating to a subject who has an average body type are used in portion discrimination, regardless of the body type of the subject, but it may be considered that variation occurs in a distance between inspection portions due to a difference between body types of subjects. Accordingly, in Embodiment 4, movement amount reference values are corrected in accordance with a body type of a subject, and the corrected movement amount reference values are used in portion discrimination.

The portion discrimination section 12 corrects a plurality of movement amount reference values in accordance with a difference between calculation results of a movement direction and a movement distance of the ultrasound probe 1 calculated by the movement amount calculation section 15, used in a case where the second inspection portion is discriminated, and movement amount reference values. Further, the plurality of corrected movement amount reference values are used for discrimination of an inspection portion that is a next inspection target subsequent to the second inspection portion.

For example, it is assumed that it is discriminated that the first inspection portion is the bladder and the second inspection portion is the heart. Further, it is assumed that a calculation result of a movement distance of the ultrasound probe 1, indicating a movement distance D of 20 cm in a case where the second inspection portion is discriminated, and a movement amount reference value of 30 cm between the bladder and the heart written in Table 1 are used. It may be considered that a proportion of the movement amount reference value to the calculation result of the movement distance of the ultrasound probe 1 between the bladder and the heart has the same value as a proportion of a movement amount reference value to a calculation result of a movement distance of the ultrasound probe 1 between different plurality of inspection portions. Thus, the proportion of the movement amount reference value to the calculation result of the movement distance of the ultrasound probe 1 between the bladder and the heart, that is, each movement amount reference value is multiplied by 2/3 for correction.

In this way, by correcting movement amount reference values in accordance with body types of subjects and using the result for discrimination of an inspection portion that is a next inspection target subsequent to the second inspection portion, it is possible to enhance discrimination accuracy.

### Embodiment 5

In Embodiments 1 to 4, movement amount reference values are used for portion discrimination regardless of subjects, but in Embodiment 5, subject reference values relating to a movement direction and a movement distance of the ultrasound probe 1 for each subject are used for portion discrimination.

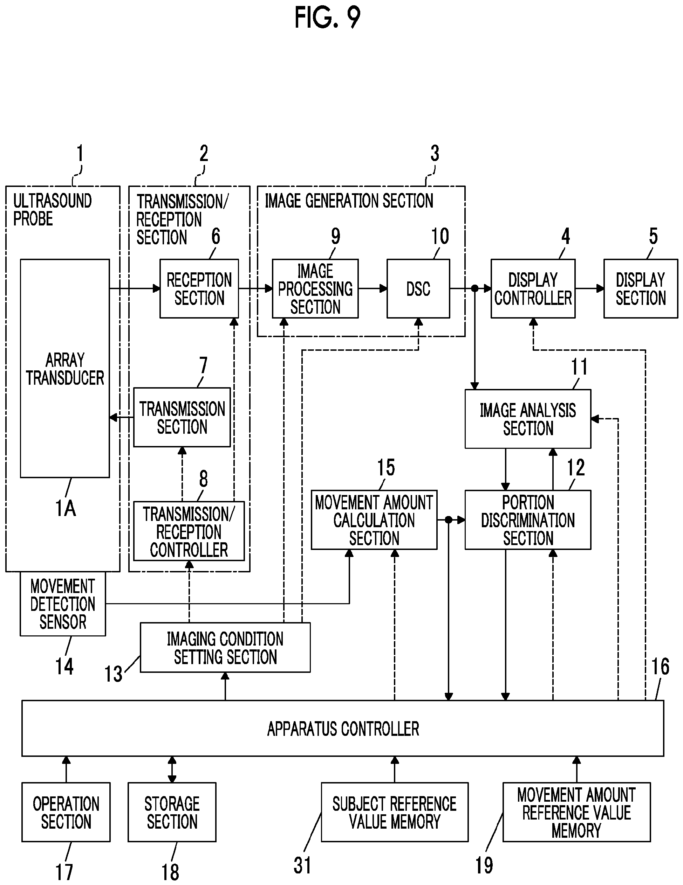

Fig. 9 shows a configuration of an ultrasound diagnostic apparatus according to Embodiment 5. The ultrasound diagnostic apparatus according to Embodiment 5 further comprises a subject reference value memory 31, in the configuration of the ultrasound diagnostic apparatus according to Embodiment 1 shown in Fig. 1, and the subject reference value memory 31 is connected to the apparatus controller 16.

The subject reference value memory 31 stores in advance subject reference values relating to a movement direction and a movement distance of the ultrasound probe 1 in a case where the ultrasound probe 1 is moved between a plurality of inspection portions for each subject. For example, the subject reference value memory 31 stores movement directions and movement distances of the ultrasound probe 1 in the past inspection for each subject.

In order to use subject reference values, for example, an operator inputs information on a subject through the operation section 17. On the basis of the input information, the portion discrimination section 12 reads out subject reference values relating to the subject from the subject reference value memory 31. Further, the read-out subject reference values are compared with calculation results of a movement direction and a movement distance of the ultrasound probe 1 calculated by the movement amount calculation section 15. Thus, it is possible to discriminate a second inspection portion in accordance with the subject, and thus, it is possible to enhance the accuracy of portion discrimination.

In a case where the subject reference values corresponding to the information on the input subject are not stored in the subject reference value memory 31, subject reference values of a different subject that is common in height, weight, gender, and the like may be used. Alternatively, movement amount reference values stored in the movement amount reference value memory 19 may be used.

### Embodiment 6

In Embodiments 1 to 5, start and termination of calculation of a movement amount of the ultrasound probe 1 is determined using an acceleration detected by the movement detection sensor 14, but in Embodiment 6, start and termination of calculation of a movement amount of the ultrasound probe 1 is determined on the basis of a determination result of whether the ultrasound probe 1 is in an air radiation state or a contact state with respect to a subject.

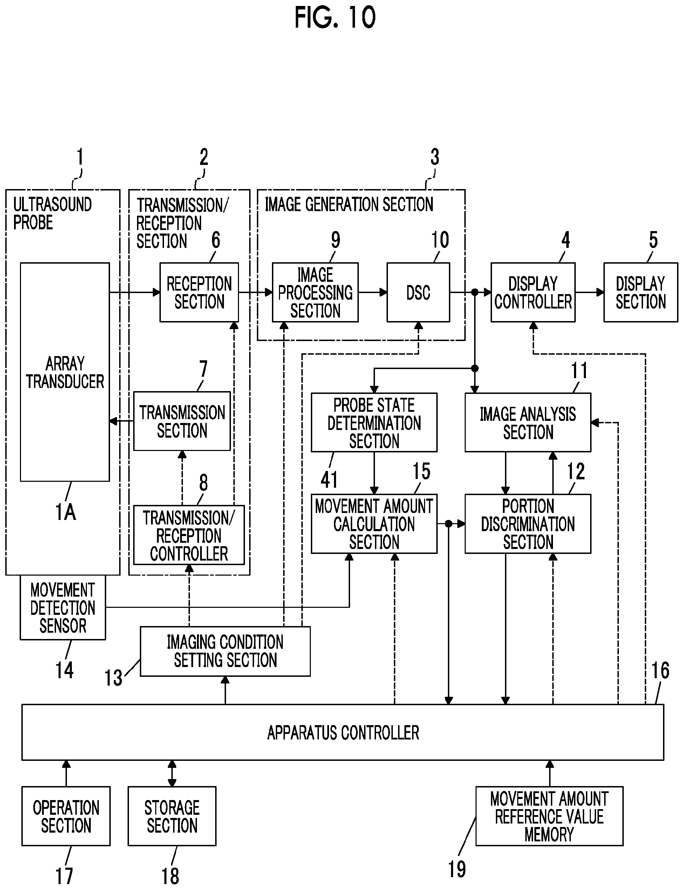

Fig. 10 shows a configuration of an ultrasound diagnostic apparatus according to Embodiment 6. The ultrasound diagnostic apparatus according to Embodiment 6 further comprises a probe state determination section 41, in the configuration of the ultrasound diagnostic apparatus according to Embodiment 1 shown in Fig. 1, and the probe state determination section 41 is connected to the DSC 10 of the image generation section 3 and is also connected to the movement amount calculation section 15.

The probe state determination section 41 determines whether the ultrasound probe 1 is in a contact state where the ultrasound probe 1 is in contact with a body surface of a subject to radiate ultrasound into the body of the subject or in an air radiation state where the ultrasound probe 1 is spaced from the body surface of the subject to radiate ultrasound in the air, using an ultrasound image signal output from the DSC 10 of the image generation section 3. Specifically, the probe state determination section 41 determines whether the ultrasound probe 1 is in the contact state with respect to the subject or in the air radiation state according to whether the presence of a structure is detected in the ultrasound image output from the DSC 10 of the image generation section 3. Further, the probe state determination section 41 outputs the determination result to the movement amount calculation section 15.

In a case where the presence of the structure is not detected in the ultrasound image, the probe state determination section 41 determines that the ultrasound probe 1 transitions from the contact state with respect to the subject to the air radiation state. Further, the probe state determination section 41 considers that the ultrasound probe 1 is spaced from the subject and is being moved from the first inspection portion to the second inspection portion, and outputs the determination result to the movement amount calculation section 15. The movement amount calculation section 15 starts calculation of a movement amount of the ultrasound probe 1, on the basis of the determination result.

Further, in a case where the presence of the structure is detected in the ultrasound image, the probe state determination section 41 determines that the ultrasound probe 1 transitions from the air radiation state to the contact state with respect to the subject. Further, the probe state determination section 41 considers that the ultrasound probe 1 completes movement and is in contact with the subject, and outputs the determination result to the movement amount calculation section 15. The movement amount calculation section 15 terminates calculation of a movement amount of the ultrasound probe 1, on the basis of the determination result.

In this way, by determining whether the ultrasound probe 1 is in an air radiation state or in a contact state with respect to a subject, and determining whether the ultrasound probe 1 is being moved between inspection portions, even in a case where a sensor other than an acceleration sensor is attached to the ultrasound probe 1, it is possible to determine start and termination of calculation of a movement amount of the ultrasound probe 1.

### Explanation of References

1: ultrasound probe
1A: array transducer
2: transmission/reception section
3: image generation section
4: display controller
5: display section
6: reception section
7: transmission section
8: transmission/reception controller
9: image processing section
10: DSC
11: image analysis section
12: portion discrimination section
13: imaging condition setting section
14: movement detection sensor
15: movement amount calculation section
16: apparatus controller
17: operation section
18: storage section
19: movement amount reference value memory
20: amplification section
21: A/D conversion section
22: beam former
23: signal processing section
31: subject reference value memory
41: probe state determination section
X: direction
D: distance
A: angle

## Claims

1. An ultrasound diagnostic apparatus that sequentially inspects a plurality of inspection portions of a subject, comprising:
an ultrasound probe (1);
an imaging section (3) that performs transmission and reception of an ultrasound beam between the ultrasound probe and the subject and generates an ultrasound image on the basis of a reception signal output from the ultrasound probe;
an image analysis section (11) that performs image analysis using the ultrasound image generated by the imaging section;
an acceleration sensor (14) that is attached to the ultrasound probe and detects an acceleration of the ultrasound probe to output the movement as a detection signal;
a movement amount reference value memory (19) in which a plurality of movement amount reference values relating to the movement direction and the movement distance of the ultrasound probe in a case where the ultrasound probe is moved between the plurality of inspection portions are stored in advance;
a movement amount calculation section (15) that determines whether the acceleration detected by the acceleration sensor is greater than or equal to a predetermined threshold value or not, continues a calculation of a movement direction and a movement distance of the ultrasound probe using the detection signal output from the acceleration sensor in a case where it is determined that the acceleration detected by the acceleration sensor is greater than or equal to the predetermined threshold value and terminates the calculation of the movement direction and the movement distance of the ultrasound probe in a case where it is determined that the acceleration detected by the acceleration sensor is smaller than the predetermined threshold value, and obtains calculation results of the movement direction and the movement distance of the ultrasound probe moved from a first inspection portion where inspection is terminated among the plurality of inspection portions to a second inspection portion that is the next inspection target;
a portion discrimination section (12) that reads out the plurality of movement amount reference values from the movement amount reference value memory, sets separation allowances with respect to a plurality of read-out movement amount reference values, compares each of the plurality of read-out movement amount reference values with the obtained calculation results of the movement direction and the movement distance of the ultrasound probe, and discriminates the second inspection portion on the basis of the comparison result and an image analysis result in the image analysis section with respect to an inspection portion where the obtained calculation results are within ranges of the separation allowances; and
an imaging condition setting section (13) configured to set an imaging condition corresponding to the second inspection portion discriminated by the portion discrimination section,
wherein the imaging section (3) generates the ultrasound image in accordance with the imaging condition set by the imaging condition setting section.

2. The ultrasound diagnostic apparatus according to claim 1,
wherein the portion discrimination section (12) integrates the image analysis result in the image analysis section and the movement direction and the movement distance calculated by the movement amount calculation section (15) to discriminate the second inspection portion.

3. The ultrasound diagnostic apparatus according to claim 1,
wherein the portion discrimination section (12) narrows down the plurality of inspection portions that are targets of the image analysis, on the basis of the movement direction and the movement distance calculated by the movement amount calculation section (15),
wherein the image analysis section (11) performs the image analysis with respect to the inspection portions narrowed down by the portion discrimination section, and
wherein the portion discrimination section (12) discriminates the second inspection portion using the image analysis result in the image analysis section (11).

4. The ultrasound diagnostic apparatus according to claim 1 or 3,
wherein the portion discrimination section (12) determines an analysis order for performing the image analysis with respect to the plurality of inspection portions, on the basis of the movement direction and the movement distance calculated by the movement amount calculation section (15),
wherein the image analysis section (11) sequentially performs the image analysis with respect to the plurality of inspection portions in accordance with the analysis order determined by the portion discrimination section (12), and
wherein the portion discrimination section (12) discriminates the second inspection portion using the image analysis result in the image analysis section (11).

5. The ultrasound diagnostic apparatus according to claim 1-4,
wherein the portion discrimination section (12) corrects the plurality of movement amount reference values in accordance with differences between the movement direction and the movement distance calculated by the movement amount calculation section (15) and used when the second inspection portion is discriminated and the movement amount reference values used when the second inspection portion is discriminated, and uses the plurality of corrected movement amount reference values in discriminating an inspection portion that becomes a next inspection target subsequent to the second inspection portion.

6. The ultrasound diagnostic apparatus according to any one of claims 1 to 5, further comprising:
a subject reference value memory (31) in which a plurality of subj ect reference values relating to the movement direction and the movement distance in a case where the ultrasound probe (1) is moved between the plurality of inspection portions for each subject are stored in advance,
wherein the portion discrimination section (12) reads out the subject reference values corresponding the subject from the subject reference value memory (31), compares the read-out subject reference values with the movement direction and the movement distance calculated by the movement amount calculation section (15), and discriminates the second inspection portion on the basis of the comparison result and the image analysis result in the image analysis section (11).

7. The ultrasound diagnostic apparatus according to any one of claims 1 to 6, further comprising:
a probe state determination section (41) that determines whether the ultrasound probe (1) is in an air radiation state or in a contact state with respect to the subject,
wherein the movement amount calculation section (15) calculates the movement direction and the movement distance of the ultrasound probe (1) from a time when it is determined by the probe state determination section (41) that the ultrasound probe transitions from the contact state with respect to the subject to the air radiation state to a time when it is determined by the probe state determination section that the ultrasound probe transitions from the air radiation state to the contact state with respect to the subject.

8. The ultrasound diagnostic apparatus according to any one of claims 1 to 7, wherein a gyro sensor, a magnetic sensor or a GPS sensor or other sensors capable of detecting a movement are used in combination with the acceleration sensor to output the movement as the detection signal.

9. A control method of an ultrasound diagnostic apparatus that sequentially inspects a plurality of inspection portions of a subject, comprising:
performing transmission and reception of an ultrasound beam between an ultrasound probe (1) and the subject, and generating an ultrasound image on the basis of a reception signal output from the ultrasound probe;
detecting an acceleration of the ultrasound probe to output the movement as a detection signal;
storing in advance a plurality of movement amount reference values relating to the movement direction and the movement distance of the ultrasound probe in a case where the ultrasound probe is moved between the plurality of inspection portions;
determining whether the detected acceleration is greater than or equal to a predetermined threshold value,
continuing a calculation of a movement direction and a movement distance of the ultrasound probe using the detection signal in a case where it is determined that the detected acceleration is equal to or greater than the predetermined threshold value,
terminating the calculation of the movement direction and the movement distance of the ultrasound probe in a case where it is determined that the detected acceleration is smaller than the predetermined threshold value, and obtaining calculation results of the movement direction and the movement distance of the ultrasound probe moved from a first inspection portion where inspection is terminated among the plurality of inspection portions to a second inspection portion that is the next inspection target;
reading out the plurality of movement amount reference values;
setting separation allowances with respect to a plurality of read-out movement amount reference values;
performing image analysis using the generated ultrasound image with respect to an inspection portion where the calculation results of the movement direction and the movement distance of the ultrasound probe are within ranges of the separation allowances;
comparing each of the plurality of read-out movement amount reference values with the calculation results;
discriminating the second inspection portion on the basis of an image analysis result and the comparison result; and
setting an imaging condition corresponding to the discriminated second inspection portion,
wherein the ultrasound image is generated in accordance with the imaging condition set.

## Patentansprüche

1. Ultraschalldiagnosevorrichtung, die sequentiell eine Mehrzahl von Prüfabschnitten eines Subjekts prüft, umfassend:
eine Ultraschallsonde (1);
einen Bildgebungsabschnitt (3), der ein Senden und ein Empfangen eines Ultraschallstrahls zwischen der Ultraschallsonde und dem Subjekt ausführt und ein Ultraschallbild auf der Grundlage eines von der Ultraschallsonde ausgegebenen Empfangssignals erzeugt;
einen Bildanalyseabschnitt (11), der eine Bildanalyse mit Hilfe des von dem Bildgebungsabschnitt erzeugten Ultraschallbilds ausführt;
einen Beschleunigungssensor (14), der an der Ultraschallsonde befestigt ist und eine Beschleunigung der Ultraschallsonde zum Ausgeben der Bewegung als Detektorsignal detektiert;
einen Bewegungshub-Referenzwertspeichert (19), in dem eine Mehrzahl von Bewegungshub-Referenzwerten bezüglich der Bewegungsrichtung und des Bewegungshubs der Ultraschallsonde für den Fall vorab gespeichert sind, dass die Ultraschallsonde zwischen den mehreren Prüfabschnitten bewegt wird;
einen Bewegungshub-Berechnungsabschnitt (15), der feststellt, ob die von dem Beschleunigungssensor detektierte Beschleunigung größer oder gleich einem vorbestimmten Schwellenwert ist oder nicht, eine Berechnung einer Bewegungsrichtung und eines Bewegungshubs der Ultraschallsonde anhand des von dem Beschleunigungssensors ausgebebenen Detektorsignals fortsetzt, falls festgestellt wird, dass die von dem Beschleunigungssensor detektierte Beschleunigung größer oder gleich dem vorbestimmten Schwellenwert ist, und die Berechnung der Bewegungsrichtung und des Bewegungshubs der Ultraschallsonde dann beendet, wenn festgestellt wird, dass die von dem Beschleunigungssensor detektierte Beschleunigung kleiner ist als der vorbestimmte Schwellenwert, und Bewegungsergebnisse der Bewegungsrichtung und des Bewegungshubs der Ultraschallsonde erhält, die von einem ersten Prüfabschnitt, bei dem die Prüfung unter den mehreren Prüfabschnitten abgeschlossen ist, zu einem zweiten Prüfabschnitt als nächstem Prüfungsziel bewegt wurde;
einen Abschnitts-Unterscheidungsabschnitt (12), der die mehreren Bewegungshub-Referenzwerte aus dem Bewegungshub-Referenzwertspeicher ausliest, Abstandstoleranzen bezüglich einer Mehrzahl von ausgelesenen Bewegungshub-Referenzwerten einstellt, jeden der mehreren ausgelesenen Bewegungshub-Referenzwerte mit den gewonnenen Berechnungsergebnissen der Bewegungsrichtung und des Bewegungshubs der Ultraschallsonde vergleicht, und den zweiten Prüfabschnitt auf der Grundlage des Vergleichsergebnisses und eines Bildanalyseergebnisses in dem Bildanalyseabschnitt in Bezug auf einen Prüfabschnitt unterscheidet, wo die erhaltenen Berechnungsergebnisse innerhalb der Bereiche der Abstandstoleranzen liegen;
einen Abbildungsbedingungs-Einstellabschnitt (13), konfiguriert zum Einstellen einer Bildgebungsbedingung entsprechend dem zweiten Prüfabschnitt, der von dem Unterscheidungsabschnitt ermittelt wurde,
wobei der Bildgebungsabschnitt (3) das Ultraschallbild nach Maßgabe der Bildgebungsbedingung erzeugt, die von dem Bildgebungsbedingungs-Einstellabschnitt eingestellt wurde.

2. Ultraschalldiagnosevorrichtung nach Anspruch 1,
bei der der Abschnitts-Unterscheidungsabschnitt (12) das Bildanalysegerät in dem Bildanalyseabschnitt integriert und die Bewegungsrichtung und der Bewegungshub, der von dem Bewegungshub-Berechnungsabschnitt (15) berechnet wurden, integriert, um den zweiten Prüfabschnitt zu unterscheiden.

3. Ultraschalldiagnosevorrichtung nach Anspruch 1,
bei der der Abschnitts-Unterscheidungsabschnitt (12) die mehreren Prüfabschnitte, die Ziele der Bildanalyse sind, auf der Grundlage der Bewegungsrichtung und des Bewegungshubs, die von dem Bewegungshub-Berechnungsabschnitt (15) berechnet wurden, eingrenzt,
wobei der Bildanalyseabschnitt (11) die Bildanalyse bezüglich der durch den Abschnitts-Unterscheidungsabschnitt eingegrenzten Prüfabschnitte ausführt, und
wobei der Abschnitts-Unterscheidungsabschnitt (12) den zweiten Prüfabschnitt mit Hilfe des Bildanalyseergebnisses in dem Bildanalyseabschnitt (11) unterscheidet.

4. Ultraschalldiagnosevorrichtung nach Anspruch 1 oder 3,
bei der der Abschnitts-Unterscheidungsabschnitt (12) eine Analysereihenfolge zum Ausführen der Bildanalyse bezüglich der mehreren Prüfabschnitte festlegt auf der Grundlage der Bewegungsrichtung und des Bewegungshubs, die von dem Bewegungshub-Berechnungsabschnitt (15) berechnet wurden,
wobei der Bildanalyseabschnitt (11) die Bildanalyse bezüglich der mehreren Prüfabschnitte gemäß der Analysereihenfolge, die von dem Abschnitts-Unterscheidungsabschnitt (12) festgelegt wurde, sequentiell ausführt, und
wobei der Abschnitts-Unterscheidungsabschnitt (12) den zweiten Prüfabschnitt anhand des Bildanalyseergebnisses in dem Bildanalyseabschnitt (11) unterscheidet.

5. Ultraschalldiagnosevorrichtung nach Anspruch 1 bis 4,
bei der der Abschnitts-Unterscheidungsabschnitt (12) die mehreren Bewegungshub-Referenzwerte nach Maßgabe von Differenzen zwischen der Bewegungsrichtung und dem Bewegungshub, die von dem Bewegungshub-Berechnungsabschnitt (15) berechnet wurden und verwendet werden, wenn der zweite Prüfabschnitt unterschieden ist und die Bewegungshub-Referenzwerte beim Unterscheiden des zweiten Prüfabschnitts verwendet werden, korrigiert und die mehreren korrigierten Bewegungshub-Referenzwerte beim Unterscheiden eines Prüfabschnitts, der das nächste Prüfziel anschließend an den zweiten Prüfabschnitt wird, verwendet.

6. Ultraschalldiagnosevorrichtung nach einem der Ansprüche 1 bis 5, weiterhin umfassend:
einen Subjekt-Referenzwertspeicher (31), in welchem eine Mehrzahl von Subjekt-Referenzwerten bezüglich der Bewegungsrichtung und dem Bewegungshub für den Fall vorab gespeichert werden, dass die Ultraschallsonde (1) zwischen den mehreren Prüfabschnitten bewegt wird,
wobei der Abschnitts-Unterscheidungsabschnitt (12) die Subjekt-Referenzwerte entsprechend dem Subjekt aus dem Subjekt-Referenzwertspeicher (31) ausliest, die ausgelesenen Subjekt-Referenzwerte vergleicht mit der Bewegungsrichtung und dem Bewegungshub, der von dem Bewegungshub-Berechnungsabschnitt (15) berechnet wurde, und den zweiten Prüfabschnitt unterscheidet auf der Grundlage des Vergleichsergebnisses und des Bildanalyseergebnisses in dem Bildanalyseabschnitt (11).

7. Ultraschalldiagnosevorrichtung nach einem der Ansprüche 1 bis 6, weiterhin umfassend:
einen Sondenzustands-Bestimmungsabschnitt (41), welcher feststellt, ob die Ultraschallsonde (1) sich in einem Luftbestrahlungszustand oder in einem Berührungszustand bezüglich des Subjekts befindet,
wobei der Bewegungshub-Berechnungsabschnitt (15) die Bewegungsrichtung und den Bewegungshub der Ultraschallsonde (1) berechnet anhand einer Zeit, zu der von dem Sondenzustands-Bestimmungsabschnitt (41) festgestellt wird, dass die Ultraschallsonde von dem Berührungszustand bezüglich des Subjekts in den Luftbestrahlungszustand übergeht, bis zu einer Zeit, zu der von dem Sondenzustands-Bestimmungsabschnitt festgestellt wird, dass die Ultraschallsonde von dem Luftbestrahlungszustand in den Berührungszustand bezüglich des Subjekts übergeht.

8. Ultraschalldiagnosevorrichtung nach einem der Ansprüche 1 bis 7,
bei der ein Gyrosensor, ein Magnetsensor oder ein GPS-Sensor oder andere Sensoren, die in der Lage sind zum Erfassen einer Bewegung, in Kombination mit dem Beschleunigungssensor eingesetzt sind zum Ausgeben der Bewegung als das Detektorsignal.

9. Steuerverfahren für eine Ultraschalldiagnosevorrichtung, die sequentiell eine Mehrzahl von Prüfabschnitten eines Subjekts prüft, umfassend:
Ausführen eines Sende- und Empfangsvorgangs eines Ultraschallstrahls zwischen einer Ultraschallsonde (1) und dem Subjekt, und Erzeugen eines Ultraschallbilds auf der Grundlage eines Empfangssignals, das von der Ultraschallsonde ausgegeben wird;
Nachweisen einer Beschleunigung der Ultraschallsonde zum Ausgeben der Bewegung als Detektorsignal;
Vorab-Speichern einer Mehrzahl von Bewegungshub-Referenzwerten bezüglich der Bewegungsrichtung und des Bewegungshubs der Ultraschallsonde für den Fall, dass die Ultraschallsonde zwischen den mehreren Prüfabschnitten bewegt wird;
Feststellen, ob die nachgewiesene Beschleunigung größer oder gleich einem vorbestimmten Schwellenwert ist,
Fortsetzen einer Berechnung einer Bewegungsrichtung und eines Bewegungshubs der Ultraschallsonde unter Verwendung des Detektorsignals für den Fall, dass festgestellt wird, dass die nachgewiesene Beschleunigung gleich oder größer ist als der vorbestimmte Schwellenwert,
Beenden der Berechnung der Bewegungsrichtung und des Bewegungshubs der Ultraschallsonde für den Fall, dass festgestellt wird, dass die nachgewiesene Beschleunigung kleiner ist als der vorbestimmte Schwellenwert, und Gewinnen von Berechnungsergebnissen der Bewegungsrichtung und des Bewegungshubs der Ultraschallsonde, die von einem ersten Prüfabschnitt, wo die Prüfung unter den mehreren Prüfabschnitten beendet ist, zu einem zweiten Prüfabschnitt bewegt wird, bei dem es sich um das nächste Prüfziel handelt;
Auslesen der mehreren Bewegungshub-Referenzwerte;
Einstellen von Abstandstoleranzen bezüglich einer Mehrzahl ausgelesener Bewegungshub-Referenzwerte;
Ausführen einer Bildanalyse unter Verwendung des erzeugten Ultraschallbilds bezüglich eines Prüfabschnitts, wo die Berechnungsergebnisse der Bewegungsrichtung und des Bewegungshubs der Ultraschallsonde innerhalb der Abstandstoleranzen liegen;
Vergleichen jedes der mehreren ausgelesenen Bewegungshub-Referenzwerte mit den Berechnungsergebnissen;
Unterscheiden des zweiten Prüfabschnitts auf der Grundlage des Bildanalyseergebnisses und des Vergleichsergebnisses; und
Einstellen einer Bildgebungsbedingung entsprechend dem unterschiedenen zweiten Prüfabschnitt,
wobei das Ultraschallbild nach Maßgabe der eingestellten Bildgebungsbedingung erzeugt wird.

## Revendications

1. Appareil de diagnostic à ultrasons, lequel inspecte de manière séquentielle une pluralité de portions d'inspection d'un sujet, comprenant :
une sonde ultrasonore (1) ;
une section d'imagerie (3), laquelle réalise une transmission et une réception d'un faisceau ultrasonore entre la sonde ultrasonore et le sujet, et génère une image échographique sur la base d'un signal de réception produit à partir de la sonde ultrasonore ;
une section d'analyse d'image (11), laquelle réalise une analyse d'image à l'aide de l'image échographique générée par la section d'imagerie ;
un capteur d'accélération (14), lequel est fixé sur la sonde ultrasonore et détecte une accélération de la sonde ultrasonore pour produire le déplacement comme signal de détection ;
une mémoire de valeurs de référence de quantité de déplacement (19), où une pluralité de valeurs de référence de quantité de déplacement liées à la direction de déplacement et la distance de déplacement de la sonde ultrasonore dans un cas où la sonde ultrasonore est déplacée entre la pluralité de portions d'inspection, sont stockées à l'avance, et
une section de calcul de quantité de déplacement (15), laquelle détermine si oui ou non l'accélération détectée par le capteur d'accélération est supérieure ou égale à une valeur seuil prédéterminée, continue un calcul d'une direction de déplacement et d'une distance de déplacement de la sonde ultrasonore à l'aide du signal de détection produit à partir du capteur d'accélération dans un cas où il est déterminé que l'accélération détectée par le capteur d'accélération est supérieure ou égale à la valeur seuil prédéterminée, et termine le calcul de la direction de déplacement et de la distance de déplacement de la sonde ultrasonore dans un cas où il est déterminé que l'accélération détectée par le capteur d'accélération est inférieure à la valeur seuil prédéterminée, et obtient des résultats de calcul de la direction de déplacement et de la distance de déplacement de la sonde ultrasonore déplacée d'une première portion d'inspection, où l'inspection est terminée, parmi la pluralité de portions d'inspection, jusqu'à une seconde portion d'inspection, laquelle est la cible d'inspection suivante ;
une section de discrimination de portion (12), laquelle lit la pluralité de valeurs de référence de quantité de déplacement dans la mémoire de valeurs de référence de quantité de déplacement, règle des marges de tolérance de séparation par rapport à une pluralité de valeurs de référence de quantité de déplacement lues, compare chaque valeur, parmi la pluralité de valeurs de référence de quantité de déplacement lues, aux résultats de calcul obtenus de la direction de déplacement et de la distance de déplacement de la sonde ultrasonore, et discrimine la seconde portion d'inspection sur la base du résultat de comparaison et d'un résultat d'analyse d'image dans la section d'analyse d'image par rapport à une portion d'inspection où les résultats de calcul obtenus sont compris dans les limites des plages des marges de tolérance de séparation, et
une section de réglage de condition d'imagerie (13), configurée pour régler une condition d'imagerie correspondant à la seconde portion d'inspection discriminée par la section de discrimination de portion,
dans lequel la section d'imagerie (3) génère l'image échographique conformément à la condition d'imagerie réglée par la section de réglage de condition d'imagerie.

2. Appareil de diagnostic à ultrasons selon la revendication 1,
dans lequel la section de discrimination de portion (12) intègre le résultat d'analyse d'image dans la section d'analyse d'image, et la quantité de déplacement et la distance de déplacement calculées par la section de calcul de quantité de déplacement (15) afin de discriminer la seconde portion d'inspection d'imagerie.

3. Appareil de diagnostic à ultrasons selon la revendication 1,
dans lequel la section de discrimination de portion (12) réduit la pluralité de portions d'inspection, lesquelles sont des cibles de l'analyse d'image, sur la base de la direction de déplacement et de la distance de déplacement calculées par la section de calcul de quantité de déplacement (15) ;
dans lequel la section d'analyse d'image (11) réalise l'analyse d'image par rapport aux portions d'inspection réduites par la section de discrimination de portion, et
dans lequel la section de discrimination de portion (12) discrimine la portion d'inspection à l'aide du résultat d'analyse d'image dans la section d'analyse d'image (11).

4. Appareil de diagnostic à ultrasons selon la revendication 1 ou 3,
dans lequel la section de discrimination de portion (12) détermine un ordre d'analyse pour réaliser l'analyse d'image par rapport à la pluralité de portions d'inspection, sur la base de la quantité de déplacement et de la distance de déplacement calculées par la section de calcul de quantité de déplacement (15) ;
dans lequel la section d'analyse d'image (11) réalise de manière séquentielle l'analyse d'image par rapport à la pluralité de portions d'inspection conformément à l'ordre d'analyse déterminé par la section de discrimination de portion (12), et
dans lequel la section de discrimination de portion (12) discrimine la seconde portion d'inspection à l'aide du résultat d'analyse d'image dans la section d'analyse d'image (11).

5. Appareil de diagnostic à ultrasons selon l'une quelconque des revendications 1 à 4,
dans lequel la section de discrimination de portion (12) corrige la pluralité de valeurs de référence de quantité de déplacement conformément aux différences entre la direction de déplacement et la distance de déplacement calculées par la section de calcul de quantité de déplacement (15) et utilisées lorsque la seconde portion d'inspection est discriminée, et les valeurs de référence de quantité de déplacement utilisées lorsque la seconde portion d'inspection est discriminée, et utilise la pluralité de valeurs de référence de quantité de déplacement corrigées pour la discrimination d'une portion d'inspection qui devient une cible d'inspection suivante, consécutive à la seconde portion d'inspection.

6. Appareil de diagnostic à ultrasons selon l'une quelconque des revendications 1 à 5, comprenant en outre :
une mémoire de valeurs de référence de sujet (31), où une pluralité de valeurs de référence de sujet liées à la direction de déplacement et la distance de déplacement dans un cas où la sonde ultrasonore (1) est déplacée entre la pluralité de portions d'inspection pour chaque sujet, sont stockées à l'avance,
dans lequel la section de discrimination de portion (12) lit les valeurs de référence de sujet correspondant au sujet dans la mémoire de valeurs de référence de sujet (31), compare les valeurs de référence de sujet lues avec la direction de déplacement et la distance de déplacement calculées par la section de calcul de quantité de déplacement (15), et discrimine la seconde portion d'inspection sur la base du résultat de comparaison et du résultat d'analyse d'image dans la section d'analyse d'image (11).

7. Appareil de diagnostic à ultrasons selon l'une quelconque des revendications 1 à 6, comprenant en outre :
une section de détermination d'état de sonde (41), laquelle détermine si oui ou non la sonde ultrasonore (1) est dans un état de rayonnement d'air ou dans un état de contact par rapport au sujet,
dans lequel la section de calcul de quantité de déplacement (15) calcule la direction de déplacement et la distance de déplacement de la sonde ultrasonore (1) d'un temps où il est déterminé par la section de détermination d'état de sonde (41) que la sonde ultrasonore présente une transition d'un état de contact par rapport au sujet à un état de rayonnement d'air à un temps où il est déterminé par la section de détermination d'état de sonde que la sonde ultrasonore présente une transition de l'état de rayonnement d'air à l'état de contact par rapport au sujet.

8. Appareil de diagnostic à ultrasons selon l'une quelconque des revendications 1 à 7,
dans lequel un capteur gyroscopique, un capteur magnétique, ou un capteur GPS, ou autres capteurs en mesure de détecter un déplacement sont utilisés en combinaison avec le capteur d'accélération pour produire le déplacement comme signal de détection.

9. Procédé de commande d'un appareil de diagnostic à ultrasons, lequel inspecte de manière séquentielle une pluralité de portions d'inspection d'un sujet, comprenant les étapes consistant à :
réaliser une transmission et une réception d'un faisceau ultrasonore entre une sonde ultrasonore (1) et le sujet, et générer une image échographique sur la base d'un signal de réception produit à partir de la sonde ultrasonore ;
détecter une accélération de la sonde ultrasonore pour produire le déplacement comme signal de détection ;
stocker à l'avance une pluralité de valeurs de référence de quantité de déplacement liées à la direction de déplacement et la distance de déplacement de la sonde ultrasonore dans un cas où la sonde ultrasonore est déplacée entre la pluralité de portions d'inspection ;
déterminer si oui ou non l'accélération détectée est supérieure ou égale à une valeur seuil prédéterminée ;
continuer un calcul d'une direction de déplacement et d'une distance de déplacement de la sonde ultrasonore à l'aide du signal de détection dans un cas où il est déterminé que l'accélération détectée est supérieure ou égale à la valeur seuil prédéterminée ;
terminer le calcul de la direction de déplacement et de la distance de déplacement de la sonde ultrasonore dans un cas où il est déterminé que l'accélération détectée est inférieure à la valeur seuil prédéterminée, et obtenir des résultats de calcul de la direction de déplacement et de la distance de déplacement de la sonde ultrasonore déplacée à partir d'une première portion d'inspection, où l'inspection est terminée parmi la pluralité de portions d'inspection, jusqu'à une seconde portion d'inspection, laquelle est la cible d'inspection suivante ;
lire la pluralité de valeurs de référence de quantité de déplacement ;
régler des marges de tolérance de séparation par rapport à une pluralité de valeurs de référence de quantité de déplacement lues ;
réaliser une analyse d'image à l'aide de l'image échographique générée par rapport à une portion d'inspection où les résultats de calcul de la direction de déplacement et de la distance de déplacement de la sonde ultrasonore sont compris dans les limites des plages des marges de tolérance de séparation ;
comparer chaque valeur parmi la pluralité de valeurs de référence de quantité de déplacement lues aux résultats de calcul ;
discriminer la seconde portion d'inspection sur la base d'un résultat d'analyse d'image et du résultat de comparaison, et
régler une condition d'imagerie correspondant à la seconde portion d'inspection discriminée,
dans lequel l'image échographique est générée conformément à la condition d'imagerie réglée.
